# EUROPEAN PATENT SPECIFICATION

(11) **EP 2 052 668 B1**
(45) Date of publication and mention of the grant of the patent: **11.01.2017**
(21) Application number: 07743657.4
(22) Date of filing: 18.05.2007
(51) Int. Cl.: A61B 1/00, A61B 1/012, A61B 1/015, G02B 23/24, A61B 90/00

(54) **ENDOSCOPE AND ENDOSCOPE INSPECTION METHOD**
ENDOSKOP UND ENDOSKOPINSPEKTIONSVERFAHREN
ENDOSCOPE ET MÉTHODE D'INSPECTION PAR ENDOSCOPE

(30) Priority: 31.05.2006 JP 2006152597
(43) Date of publication of application: 29.04.2009
(73) Proprietor: Olympus Corporation, Tokyo 192-8507 (JP)
(72) Inventor: DOGUCHI, Nobuyuki, Hachioji-shi, Tokyo 192-8507 (JP); ICHIMURA, Hironobu, Hachioji-shi, Tokyo 192-8507 (JP); TAKATO, Hideyasu, Hachioji-shi, Tokyo 192-8507 (JP); NOGUCHI, Azusa, Hachioji-shi, Tokyo 192-8507 (JP); GONO, Kazuhiro, Hachioji-shi, Tokyo 192-8507 (JP); MIYAKE, Kiyoshi, Hachioji-shi, Tokyo 192-8507 (JP)
(74) Representative: von Hellfeld, Axel
(86) International application number: PCT/JP2007/060222
(87) International publication number: WO 2007/138888

(56) References cited:
- JP-A- 2001 292 959
- JP-A- 2003 190 076
- JP-A- 2003 290 125
- JP-A- 2003 290 127
- JP-A- 2004 173 768
- JP-A- 2005 080 953
- JP-A- 2005 168 927
- JP-A- 2005 253 873
- JP-A- 2005 329 094
- JP-A- 2006 325 785
- JP-A- 2006 325 867
- JP-A- 2007 151 873
- US-A1- 2001 049 509
- US-A1- 2003 187 319
- US-A1- 2003 187 349

## Description

### Technical Field

The present invention relates to an endoscope capable of marking a body wall or the like which is a subject by an endoscope.

### Background Art

Conventionally, an endoscope having an observation portion capable of a magnification observation of a subject by bringing a distal end of the observation portion into contact with the subject is disclosed in Japanese Patent Application Laid-Open Publication No. 2004-350940 . An insertion distal end portion of the endoscope is provided with a normal observation portion having a normal magnification and a magnification observation portion that can be projected from and retracted into a distal end surface of an insertion portion and has a high magnification. According to the endoscope, for example, after the normal observation portion searches for a lesion area by an observation, the magnification observation portion can perform a magnification observation or a magnification shooting of the lesion area.

In an endoscope disclosed in Japanese Patent Application Laid-Open Publication No. 2003-135377 , an observation window is provided at an insertion distal end portion and two treatment instrument insertion passages for guiding a treatment instrument are provided in an insertion portion. In the endoscope, grasping forceps projected from one of the insertion passages pick up and raise a lesion area, and a root portion of the lesion area is observed with the observation window being separated by an appropriate distance. A heat probe is projected from the other insertion passage in the observation state to mark the root portion of the lesion area.

According to the endoscope in Japanese Patent Application Laid-Open Publication No. 2004-350940 , a location of an observation area observed by the magnification observation portion can be roughly recognized by the normal observation portion, but the observation area cannot be recognized again after a magnification observation.

Also, according to the endoscope in Japanese Patent Application Laid-Open Publication No. 2003-135377 , a lesion area to be observed can be recognized even after an observation by marking the lesion area to be observed. However, this marking onto the lesion area is not an appropriate marking onto an area close to a very small area magnified and observed, but only a rough marking or marking onto an area of interest across a wide range. Further, since a method for making with an area apart from an area to be marked being raised is applied, it is not possible to appropriately mark an observation area with the magnification observation portion being in contact with the observation area.

US 2003/187349 A1 concerns a sentinel lymph node detecting system that comprises an inserting portion with a small diameter which can be inserted into the body cavity, an X-ray detecting unit for two-dimensionally detecting X-rays, and an optical imaging unit for taking visible-light images, which are mounted to the tip of the inserting portion. The X-ray detecting unit and the optical imaging unit are disposed along the longitudinal direction of the inserting portion, and are closely disposed one to another so as to observe in the same direction. A radioactive tracer accumulated in sentinel lymph nodes is detected by the X-ray detecting unit.

US 2003/187319 A1 concerns a sentinel lymph node detecting apparatus that comprises an endoscope, a visible-light CCU and infrared CCU, which are detachably connected to the endoscope, a superimposing circuit for superimposing the image output from the infrared CCU on the image output from the visible-light CCU, a monitor for displaying the image superimposed by the superimposing circuit, and a fluctuating magnetic field generating device for generating a fluctuating magnetic field for vibrating ferrofluid which has been accumulated in sentinel lymph nodes as a tracer beforehand, so as to heat the ferrofluid. A computer color-enhanced infrared image obtained by an infrared sensor is superimposed on an endoscope image obtained by visible-light CCD, and the synthesized endoscope infrared image is displayed on a display screen of the monitor.

US 2001/049509 A1 concerns an endoscopic treatment system for executing at least one of localized injection through mucous membrane, peeling off and cutting off of mucous membrane. This system has an endoscope, which has an insertion section provided with at least one channel extending therein. The system also has a plurality of treatment tools including a syringe needle for executing localized injection through a portion of mucous membrane, forceps for gripping a portion of mucous membrane, and at least one knife for executing at least one of peeling off and cutting off of mucous membrane. Further, this system includes an endoscopic guide tube to be inserted into the esophagus, which has a main channel for inserting therein the endoscope, and at least one sub channel for inserting therein each of the treatment tools.

The present invention has been made to solve the problems mentioned above and has an object to provide an endoscope capable of reliably marking an observation area.

### Disclosure of Invention

### Means for Solving the Problem

An endoscope of the present invention comprises the features of claim 1.

### Brief Description of the Drawings

Fig. 1 is a diagram showing an entire configuration of an endoscope observation apparatus to which an endoscope of a first embodiment of the present invention is applied;
Fig. 2 is an enlarged sectional view showing a distal end of an insertion portion of the endoscope in Fig. 1, which shows a state in which a high magnification observation probe is projected and is in contact with a body wall which is an observation area, and is observing an area of interest;
Fig. 3 is a view on arrow A in Fig. 2, which shows an arrangement of an objective lens system, the high magnification observation probe and illumination lenses of a distal end surface of the endoscope insertion portion;
Fig. 4 is an enlarged view of a distal end surface of the high magnification observation probe in Fig. 3;
Fig. 5A is a perspective view showing a state of a preparation operation for observing and marking by the high magnification observation probe in Fig. 3;
Fig. 5B is a perspective view showing a state in which the high magnification observation probe has marked an area close to the area of interest;
Fig. 6 is an enlarged view of a distal end surface in a first modification of the high magnification observation probe in Fig. 3;
Fig. 7A is a diagram showing an example of marking four areas around an area of interest by the high magnification observation probe of the modification in Fig. 6;
Fig. 7B is a diagram showing an example of marking three areas around an area of interest by the high magnification observation probe of the modification in Fig. 6;
Fig. 7C is a diagram showing an example of marking two areas around an area of interest by the high magnification observation probe of the modification in Fig. 6;
Fig. 8 is a view showing an arrangement of a distal end surface in a second modification to the high magnification observation probe in Fig. 3;
Fig. 9 is a diagram showing an entire configuration of an endoscope observation apparatus to which an endoscope of a second embodiment of the present invention is applied; and
Fig. 10 is an enlarged sectional view showing a distal end of an insertion portion of the endoscope in Fig. 9, which shows a state in which the high magnification observation probe is in contact with a body wall which is an observation area, and is observing an area of interest.

### Best Mode for Carrying Out the Invention

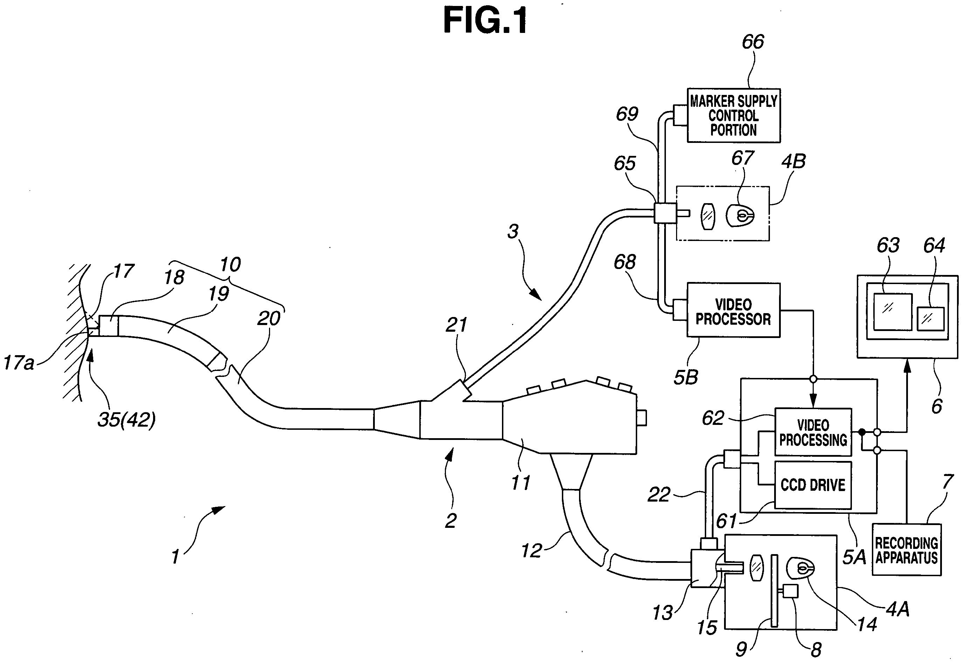

Hereinafter, embodiments of the present invention are described in detail with reference to the drawings. A first embodiment of the present invention is described with reference to Figs. 1 to 5B

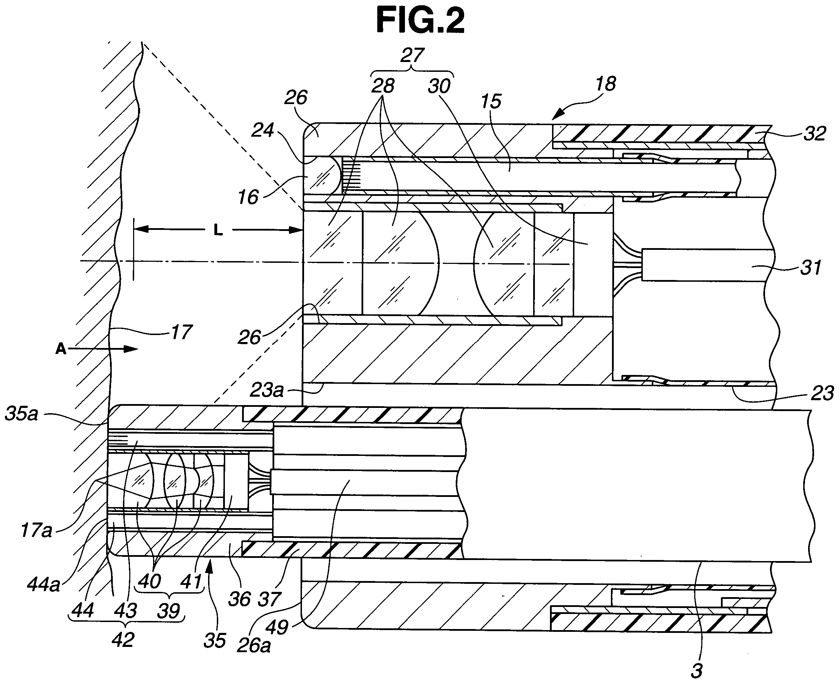

As shown in Figs. 1 and 2, an endoscope observation apparatus 1 of the first embodiment of the present invention has an endoscope 2, a high magnification observation probe 3, a light source device 4A, a video processor 5A, a monitor 6, a marker supply control portion 66, a video processor 5B, a light source device 4B and a recording apparatus 7.

The endoscope 2 has an insertion portion 10 capable of being inserted into a body cavity which is a subject. The endoscope 2 also includes first observation means. The high magnification observation probe 3 is inserted into a forceps channel (or a treatment instrument channel) 23 of the endoscope 2 so as to be movable back and forth. The high magnification observation probe 3 has a high magnification observation portion 42 which is second observation means capable of an optical high magnification observation. The light source device 4A supplies illumination light to a light guide of the endoscope 2. The video processor 5A performs signal processing for a normal observation image pickup unit 27 included in the endoscope 2. A video signal outputted from the video processor 5A is displayed on the monitor 6. The marker supply control portion 66 is marking means and supplies a marking agent (or a marker) to the high magnification observation probe 3. The video processor 5B performs signal processing for a high magnification image pickup unit 39 provided to the high magnification observation probe 3. The light source device 4B supplies illumination light to a light guide of the high magnification observation probe 3. The recording apparatus 7 records the video signal outputted to the monitor 6.

The endoscope 2 has the elongated flexible insertion portion 10 including the image pickup unit 27 which is the first observation means having a normal magnification at a distal end portion, an operation portion 11 provided at a rear end of the insertion portion 10, and a universal cord 12 extending from a side portion of the operation portion 11. A connector 13 provided at a proximal end portion of the universal cord 12 is connected to the light source device 4A detachably.

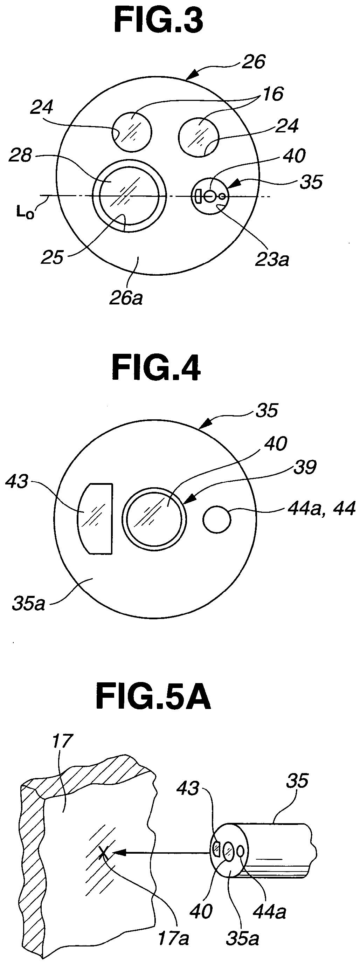

The light source device 4A includes a lamp 14 producing white light. The white light from the lamp 14 is converted into frame sequential light through a rotating filter 9 rotated by a motor 8, and to which red, green and blue transmission filters are attached. The light passing through each of the color transmission filters is condensed by a lens and enters as illumination light into a light guide 15 of a light guide base portion projecting from the connector 13. The illumination light is transmitted by the light guide 15, goes out from a distal end surface of the insertion portion 10 through illumination lenses 16 (see Fig. 3), and illuminates an observation area 17 such as a diseased part.

The light source device 4B includes a lamp 67 producing white light. The white light of the lamp 67 is condensed by a lens and enters into a light guide of the high magnification observation probe 3. The white light goes out from a distal end of the high magnification observation probe 3 to an area of interest 17a in the observation area 17 which is a subject (body wall).

The insertion portion 10 has a rigid distal end portion 18, a bendable bending portion 19 provided at a rear end of the distal end portion 18, and a long flexible portion 20 extending from a rear end of the bending portion 19 to a front end of the operation portion 11. The bending portion 19 can be bent in any direction of up and down, left and right by operating a bending knob (not shown) provided at the operation portion 11.

A distal end portion main body 26 configuring the distal end portion 18 in the insertion portion 10 is provided with two illumination windows 24 of an illumination optical system having the illumination lenses 16 arranged at a distal end side of the light guide 15, and a normal observation objective lens system 28 being held to an observation window (image pickup window) 25 provided adjacent to the illumination windows 24 by a lens frame.

Further, the image pickup unit 27 (Fig. 2) has a configuration in which, for example, a CCD 30 which is a charge coupled device is arranged as a solid image pickup device at a image formation location behind the objective lens system 28. The CCD 30 is image pickup means for a normal observation and for photoelectrically converting a formed optical image. A front surface of the CCD 30 is provided with a cover glass and an optical filter.

An insertion opening 21 for a treatment instrument is provided close to the front end of the operation portion 11 and a treatment instrument, the high magnification observation probe 3 or the like can be inserted therein. The insertion opening 21 for a treatment instrument communicates therein with the forceps channel 23 (see Fig. 2) provided along a longitudinal direction of the insertion portion 10.

A hole portion for a channel communicating with a flexible tube forming the forceps channel 23 is formed in the distal end portion main body 26. A distal end portion 35 of the high magnification observation probe 3 inserted into the forceps channel 23 can be freely projected from and retracted into a distal end opening portion 23a of the forceps channel 23. The distal end portion 35 of the high magnification observation probe 3 includes the high magnification observation portion 42 having the high magnification image pickup unit 39, a light guide 43 and a marker supply pipeline 44.

A bending piece configuring an extreme tip of the bending portion 19 is fixed at a rear end of the distal end portion main body 26. An outside of the bending piece is water-tightly covered with an exterior member 32 made of a rubber tube or the like having plenty of bending property.

As shown in Fig. 3, a distal end surface 26a of the distal end portion main body 26 is provided with the observation window 25 for a normal observation, the illumination windows 24, and the distal end opening portion 23a of the forceps channel 23, from which the distal end portion 35 of the high magnification observation probe 3 can be projected. The observation window 25 and the distal end opening portion 23a of the forceps channel 23 are located on a straight line L₀ with a predetermined distance apart from each other. The illumination windows 24 are located close to the observation window 25.

As shown in Fig. 4, a distal end surface 35a which is a contact portion in the distal end portion 35 of the high magnification observation probe 3 is provided with an objective lens system 40, which is located in a center, of the high magnification observation unit 39, the light guide 43 and an exhaust port 44a of the marker supply pipeline 44. The light guide 43 and the exhaust port 44a of the marker supply pipeline 44 are respectively located at least one side of right and left sides close to the objective lens system 40.

If it is desired to locally observe the area of interest 17a in the observation area 17 which is a subject such as a biological mucus membrane (body wall) with a high magnification, the high magnification observation probe 3 projects the distal end portion 35 from the distal end opening portion 23a of the forceps channel 23 as shown in Figs. 1 and 2, and brings the distal end surface (observation window portion) 35a into contact with a surface of the area of interest 17a. In this contact state, the distal end portion 35 is held at a predetermined location so that a histological microscopic structure in the area of interest 17a can be stably observed with a high magnification through the objective lens system 40 of the distal end portion 35.

In this observation state, an area that can be observed by the high magnification observation probe 3 is within a visual field range of a normal observation of an endoscope 2 side.

A detailed structure around the distal end portion 35 of the high magnification observation probe 3 is described later with Fig. 2 or the like.

A distal end of a signal cable 31 is connected to the CCD 30 of the image pickup unit 27 shown in Fig. 2. A rear end side of the signal cable 31 is connected to a connector bracket in a side portion of the connector 13 and connected to the video processor 5A detachably through a signal cable 22 connected to the connector bracket.

The video processor 5A includes a CCD drive circuit 61 and a video processing circuit 62. The CCD drive circuit 61 generates a CCD drive signal driving the CCD 30. The video processing circuit 62 performs signal processing to an image pickup signal outputted from the CCD 30 by applying the CCD drive signal, and generates a video signal.

The video signal generated by the video processing circuit 62 is outputted to the monitor 6 and displayed as an endoscope image by a normal observation on a normal observation image displaying area 63 of the monitor 6.

A distal end of a signal cable 49 is connected to a CCD 41 of the high magnification image pickup unit 39 side. A rear end side of the signal cable 49 is connected detachably to the video processor 5B through, for example, a cable 68 extending from a connector portion 65.

Similarly to the video processor 5A, the video processor 5B includes a CCD drive circuit and a video processing circuit. A video signal outputted from the video processor 5B and corresponding to an image pickup signal picked up and obtained at the CCD 41 is inputted to the video processing circuit 62 of the video processor 5A.

In the present embodiment, the video processing circuit 62 performs a process for generating a video signal under frame sequential illumination. On the other hand, the video processing circuit of the video processor 5B performs signal processing for generating a video signal corresponding to an image pickup signal of the CCD 41 under white light illumination. Then, the generated video signal is outputted from the video processor 5B to the video processing circuit 62 of the video processor 5A. In addition to the normal observation video signal, a high magnification observation video signal outputted from the video processor 5B is inputted to the video processor 5A and outputted to the monitor 6 through a not-shown mixer in the video processor 5A. Then, a high magnification (enlargement) observation image by the high magnification observation probe 3 is displayed on a high magnification observation image displaying area 64 adjacent to the normal observation image displaying area 63 of the monitor 6.

The distal end portion 35 of the high magnification observation probe 3 is formed with a rigid thin cylinder 36 shown in Fig. 2 and having a light blocking property. A distal end of a flexible sheath (flexible tube) 37 is water-tightly fixed to a rear end of the cylinder 36 so that a flexible insertion portion capable of being inserted into the forceps channel 23 is formed.

A hollow portion of the cylinder 36 includes the high magnification image pickup unit 39 capable of a high magnification observation as observation means, the light guide 43 for illumination, and the marker supply pipeline 44 having the marker exhaust port 44a. The light guide 43 for illumination and the marker supply pipeline 44 are included along the image pickup unit 39.

The high magnification image pickup unit 39 is provided in a center of the cylinder 36 and has the high magnification objective lens system 40 attached to a lens frame, an optical filter, and the CCD 41 which is a solid image pickup device fixed at a image formation location behind the high magnification objective lens system 40 and the optical filter. An observation magnification of the high magnification image pickup unit 39 is, for example, on the order of 200× to 1000x, an observation range is 1 mm × 1 mm or less, and a resolution during a high magnification (enlargement) observation is 5 µm or less. That is, the high magnification image pickup unit 39 observes a very small area. Once an observed area is lost sight of, it is difficult to identify the area again without a marking.

Illumination light going out from the light guide 43 of the distal end portion 35 of the high magnification observation probe 3 enters into an inside of the area of interest 17a in the observation area 17 to be reflected. Then, an image of the area of interest 17a, which is reflected by the illumination light entering into the inside, is captured through the high magnification objective lens system 40 of the distal end portion 35.

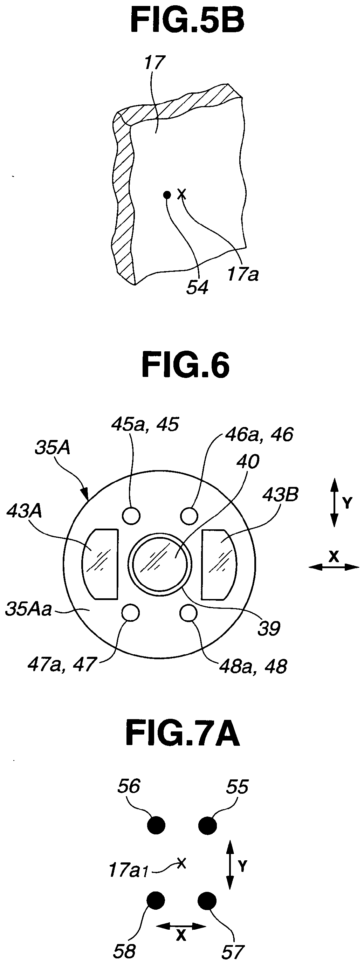

A marking agent for marking, for example, a fluid, solid, powder or gel marker including a coloring matter is supplied from the marker supply control portion 66 to the marker supply pipeline 44 through a connecting tube 69 at the time of a marking operation. The marker is discharged from the exhaust port 44a with the distal end surface 35a of the distal end portion 35 being in contact with the surface of the area of interest 17a. Because of this, a mark 54 can be applied close to the area of interest 17a as shown in Fig. 5B.

Here, an endoscopy method by the endoscope observation apparatus 1 of the present embodiment with the above configuration is described.

In the case of performing a normal observation and a high magnification observation of the observation area 17 of a biological mucus membrane, the endoscope 2 is inserted into a body cavity and the observation area 17 such as a mucus membrane is observed with a normal magnification by the normal observation image pickup unit 27 provided at the distal end portion 18 of the endoscope 2. If the area of interest 17a for which an observation of a histological microscopic structure is desired exists in the observation area 17, a tube (not shown) of a coloring matter spraying instrument inserted into the forceps channel 23 stains the area of interest 17a. After that, the tube of the coloring matter spray instrument is withdrawn from the insertion opening 21. Then, as shown in Fig. 1, the high magnification observation probe 3 is inserted into the forceps channel 23 from the insertion opening 21 and the distal end portion 35 is projected from the distal end opening portion 23a of the forceps channel 23. In the normal observation state by the endoscope 2, the distal end surface 35a of the distal end portion in the high magnification observation probe 3 is pressed against (brought into contact with) the surface of the area of interest 17a, as shown in Fig. 2.

As described above, the distal end surface 35a of the high magnification observation probe 3 is brought into close contact with the surface of the area of interest 17a with the area of interest 17a being within an observation range of the endoscope 2 so that the distal end portion 35 can be positioned without slipping out of place.

In the high magnification observation state, illumination light from the light source device 4B goes out from the light guide 43. At this time, the distal end surface 35a of the high magnification observation probe 3 is in contact with the area of interest 17a so that the illumination light going out to an inside of the area of interest 17a is scattered by an inside tissue or the like. Then, an optical image of the area of interest 17a is formed on the CCD 41 located at the image formation location of the high magnification objective lens system 40 with the distal end surface 35a being pressed.

The image formed on the CCD 41 is photoelectrically converted by the CCD 41 and converted into a video signal by the video processing circuit in the video processor 5B. Then, a high magnification observation image is displayed adjacent to an endoscope image on the monitor 6. The high magnification observation image is recorded in the recording apparatus 7 if necessary (a high magnification observation step).

If a location of the image-recorded area of interest 17a needs to be marked after recording the high magnification observation image, a marker is supplied from the marker supply control portion 66 with the distal end surface 35a of the high magnification observation probe 3 being in contact with the surface of the area of interest 17a. Because of this, the mark 54 can be applied close to the area of interest 17a as shown in Fig. 5B (a marking step).

It is desired that a projection distance of the distal end surface 35a of the high magnification observation probe 3 from the distal end surface 26a of the distal end portion main body 26 during the high magnification observation is slightly larger than a near point observation distance L with the normal observation image pickup unit 27 focusing (see Fig. 2). Setting to this state enables the normal observation by the normal observation image pickup unit 27 without changing a location of the high magnification observation probe 3 in a state capable of the high magnification observation.

The above marking operation may be performed before the high magnification observation, that is, right after bringing the distal end surface 35a of the high magnification observation probe 3 into contact with the surface of the area of interest 17a. Alternatively, the marking operation may be performed during the high magnification observation.

As described above, according to the endoscope 2 of the present embodiment, a marker is discharged from the marker exhaust port 44a of the marker supply pipeline 44 provided on the distal end surface 35a of the high magnification observation probe 3 for marking after the high magnification observation, before the observation or during the observation in a contact state of the distal end surface 35a. As a result, a location of the area of interest 17a is correctly and reliably identified, a reexamination or the like can be correctly performed, and a marking operation thereof is easy.

Additionally, since the distal end surface 35a of the high magnification observation probe 3 is in contact with the surface of the area of interest 17a at the time of marking, stable marking can be performed without slipping out of place. Further, since the marker supply pipeline 44 may be a thin tube, an outside diameter of the high magnification observation probe 3 is not large. Because of this, it is easy to insert the high magnification observation probe 3, with a thin diameter, having the marker supply pipeline 44 into the insertion portion 10.

In the case of an endoscope provided with the forceps channel 23, an endoscope observation apparatus having a marking function can be easily made by inserting the high magnification observation probe 3 having the above marking means into the forceps channel 23.

While the marking means of the present embodiment is provided in the high magnification observation probe 3, it is possible to provide the marker supply pipeline and the marker exhaust port of the marking means at the distal end portion main body 26 of the insertion portion 10. In the case of this configuration, it is necessary to bring the distal end surface 26a of the distal end portion main body 26 into contact with the observation area 17, when marking is performed. This marking can be used for identifying an observation area normally observed.

Additionally, the marking means of the present embodiment performs marking by discharging a marker such as a gel from the exhaust port 44a. However, the present invention is not limited thereto, and it is possible to perform marking by transferring the marker around the area of interest 17a.

Further, a pinhole is provided on the distal end surface of the high magnification observation probe 3 and a trace is left on a subject by projecting a needle member from the pinhole so that marking may be performed around an area of interest. Also, a material color-changed by a heating temperature is applied as the marker and a heating portion is provided on the distal end surface of the high magnification observation probe 3. Then, an area around the area of interest marked by the heating portion is heated up to be color-changed so that each area of interest 17a may be identified by the color.

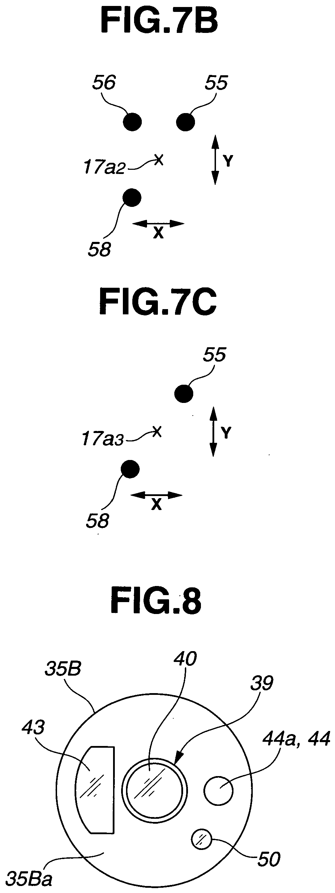

Next, a high magnification observation probe provided with a plurality of marker supply pipelines and marker exhaust ports which is marking means is described as a first modification to the high magnification observation probe 3 in the endoscope apparatus 1 of the first embodiment with reference to Figs. 6, 7A, 7B and 7C.

A distal end surface 35Aa of a contact portion in a distal end portion 35A of the high magnification observation probe in the modification is provided with the objective lens system 40 of the high magnification image pickup unit 39, illumination windows of light guides 43A and 43B, and four marker exhaust ports 45a, 46a, 47a and 48a of marker supply pipelines 45, 46, 47 and 48. The objective lens system 40 is located in a center of the distal end surface 35Aa. The illumination windows of the light guides 43A and 43B are respectively located on left and right sides (X direction in the drawing) of the objective lens system 40. Each two of the marker exhaust ports 45a, 46a, 47a and 48a are respectively located on upper and lower sides (Y direction in the drawing) of the objective lens system 40 with a predetermined distance apart from each other.

The four marker supply pipelines 45, 46, 47 and 48 are connected to a marker supply source of the marker supply control portion 66 shown in Fig. 1. The marker supply control portion 66 can selectively supply a marker to the marker supply pipelines 45, 46, 47 and 48 according to an instruction operation at the operation portion 11.

If marking an area of interest 17a1 is necessary when an observation image of the area of interest 17a1 in the observation area 17 by a high magnification observation probe 3A is recorded in the recording apparatus 7, a marker is supplied to the marker supply pipelines 45, 46, 47 and 48 by the marker supply control portion 66 with the high magnification observation probe 3A being in contact with a surface of the area of interest 17a1. Then, the marker is discharged from the marker exhaust ports 45a, 46a, 47a and 48a so that markings 55, 56, 57 and 58 are applied around the area of interest 17a1, as shown in Fig. 7A. Because of this, the area of interest 17a1 observed with a high magnification is located inside a plurality of the markings 55, 56, 57 and 58.

If marking other areas of interest 17a2 and 17a3 is necessary after that, a marker is supplied to, for example, the marker supply pipelines 45, 46 and 48 selected by the marker supply control portion 66 or to the marker supply pipelines 45 and 48 with the high magnification observation probe 3A being in contact with the surface of the area of interest 17a1 similarly to the above case. Then, the marker is discharged from the marker exhaust ports 45a, 46a and 48a to apply the markings 55, 56 and 58 around the area of interest 17a2 as shown in Fig. 7B, or the marker is discharged from the marker exhaust ports 45a and 48a to apply the markings 55 and 58 as shown in Fig. 7C. Also in this case, the area of interest 17a2 observed with a high magnification is located inside the markings 55, 56 and 58, and the area of interest 17a3 observed with a high magnification is located inside the markings 55 and 58.

The different numbers of markings shown in Figs. 7A, 7B and 7C corresponds to a record order of observation images recorded in the recording apparatus 7. Because of this, if an observed area of interest is further reexamined after recording the observation images, the different numbers of markings corresponding to the record order are searched for by a normal observation with the endoscope 2 and the corresponding area of interest can be reexamined.

According to an endoscope to which the high magnification observation probe 3A of the modification is applied, the number of markings can identify an observed area of interest corresponding to a recorded image by a high magnification observation, which is recorded in the recording apparatus 7 after recording observed images.

Spacing among the marker exhaust ports 45a, 46a, 47a and 48a provided on the distal end surface 35Aa of the high magnification observation probe may be the same in X and Y directions in the drawings, while different spacing among a plurality of markings can correspond an orientation of a recorded image to an orientation of the observed area of interest 17a.

As another modification about an arrangement of the mark exhaust ports provided on the distal end surface 35Aa of the high magnification observation probe 3A in the above modification, it can be proposed that a plurality of mark exhaust ports be arranged around the objective lens system 40 forming a ring shape. In this modification, a location of the observed area of interest 17a can be searched for easily.

Next, as a second modification to the high magnification observation probe 3 in the endoscope apparatus 1 of the first embodiment, a high magnification observation probe whose distal end surface is provided with an optical sensor is described.

As shown in Fig. 8, the objective lens system 40 of the high magnification image pickup unit 39 is located in a center of a distal end surface 35Ba which is a contact portion in a distal end portion 35B of a high magnification observation probe in the modification. The illumination window of the light guide 43 and the marker exhaust port 44a of the marker supply pipeline 44 are respectively located on left and right sides of the objective lens system 40. Additionally, an optical sensor 50 is located, for example, close to a side of the marker exhaust port 44a.

When the distal end surface 35Ba comes in contact with the surface of the area of interest 17a, the optical sensor 50 outputs a detection signal of the contact state. The detection signal is captured by the marker supply control portion 66 and a marker is automatically supplied from the marker supply control portion 66 to a mark supply pipeline in the contact state of the distal end surface 35Ba. The marker is discharged from the marker exhaust port 44a to mark around the area of interest 17a.

According to the high magnification observation probe of the modification, a contact of the distal end surface 35Ba to the surface of the area of interest 17a is automatically detected for performing marking. Thus, marking to the area of interest can be performed easily and reliably.

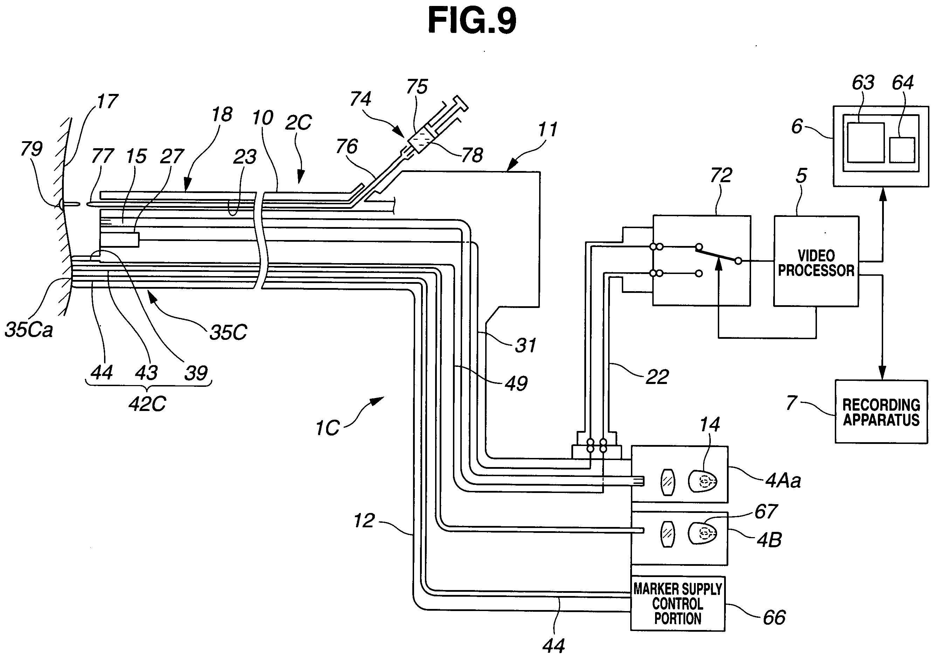

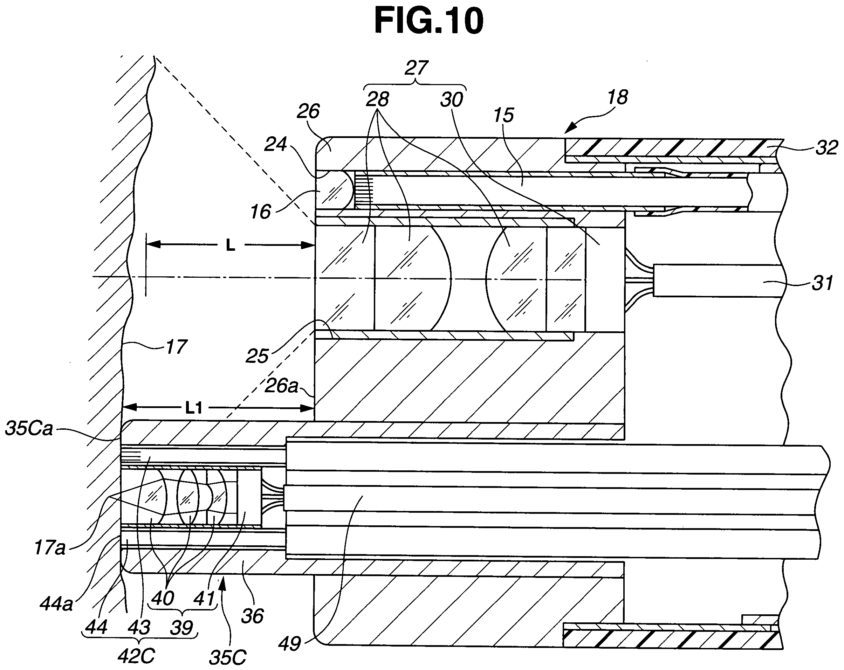

Next, an endoscope apparatus of a second embodiment of the present invention is described with reference to Figs. 9 and 10.

As shown in Figs. 9 and 10, an endoscope observation apparatus 1C of the present embodiment has an endoscope 2C, a video processor 5, a signal switching device 72, the monitor 6 displaying a video signal outputted from the video processor 5, a light source device 4Aa, the light source device 4B, the marker supply control portion 66 and the recording apparatus 7 recording the video signal outputted to the monitor 6. The endoscope 2C has an insertion portion capable of being inserted into a body cavity of a subject. The video processor 5 performs signal processing for the normal observation image pickup unit 27 included in the endoscope 2 and signal processing for the image pickup unit 39 of a high magnification observation probe portion 42C. The signal switching device 72 switches outputs of the image pickup unit 27 and the image pickup unit 39. The light source device 4Aa supplies white illumination light to a light guide of the endoscope 2. The light source device 4B supplies white illumination light to a light guide of the high magnification observation probe portion 42C. The marker supply control portion 66 is marking means for supplying a marker to the high magnification observation probe portion 42C.

The light source device 4Aa includes the lamp 14 producing white light. Illumination light being the white light of the lamp 14 is condensed by a lens to enter into the light guide 15 of the light guide base portion. The illumination light is transmitted by the light guide 15, goes out from the distal end surface of the insertion portion 10 through the illumination lenses 16 (Fig. 10), and illuminates the observation area 17 such as a diseased part.

The light source device 4B includes the lamp 67 producing white light. Illumination light being the white light of the lamp 67 is condensed by a lens, enters into the light guide 43 of the high magnification observation probe portion 42C, and goes out from a distal end of the high magnification observation probe portion 42C to the area of interest 17a in the observation area 17 which is a subject.

The signal cable 31 of the image pickup unit 27 and the signal cable 49 of the high magnification image pickup unit 39 are inserted into the insertion portion 10, the operation portion 11 and the universal cord 12 connected to the operation portion 11 of the endoscope 2C. Additionally, the signal cables 31 and 49 are inserted into the signal cable 22. Velocities of the signal cables 31 and 49 can be switched not only to a low velocity, but also to a high velocity by an analog switch in the signal switching device 72.

The endoscope 2C has the elongated flexible insertion portion 10 and the operation portion 11 provided at the rear end of the insertion portion 10. The distal end portion 18 of the insertion portion 10 is integrally provided with the normal observation image pickup unit 27 as first observation means having a normal magnification along the light guide 15 and the high magnification observation probe portion 42C as second observation means capable of an optical high magnification observation. Further, the endoscope 2C has a coloring matter spray instrument 74 inserted into the forceps channel 23 of the insertion portion 10.

As shown in Fig. 10, the image pickup unit 27 includes the objective lens system 28 having a normal magnification and the CCD 30. The signal cable 31 for driving and for transmitting an image pickup signal is connected to the CCD 30. The illumination lenses 16 are provided on the distal end side of the light guide 15. The image pickup unit 27 and the illumination lenses 16 are respectively located inside the observation window 25 and the illumination windows 24 of the distal end surface 26a of the distal end portion main body 26 in the distal end portion 18.

The high magnification observation probe portion 42C is fitted in the cylinder 36 of a distal end portion 35C and is integral with the distal end portion main body 26 of an endoscope side. A distal end surface 35Ca of the distal end portion 35C projects from the distal end surface 26a by a projection distance L1.

The high magnification observation probe portion 42C includes the high magnification image pickup unit 39 with the high magnification objective lens system 40 and the CCD 41, the light guide 43, and the marker supply pipeline 44 which is marking means. The signal cable 49 for driving and for transmitting an image pickup signal is connected to the CCD 41.

Optical axes of the objective lens system 28 in the normal observation image pickup unit 27 and the objective lens system 40 in the high magnification observation probe portion 42C are located with a predetermined distance apart from each other. An observation visual field of the high magnification observation probe portion 42C is within an observation visual field of the image pickup unit 27.

The projection distance L1 of the high magnification observation probe portion 42C is fixed to be larger than the near point distance L allowing the normal observation objective lens system 28 to perform an observation in a focus state. That is, L1 is larger than L (L < LI).

Accordingly, also in the present embodiment, a small area (almost a dot) in an observation range in a state where the normal observation image pickup unit 27 of the endoscope 2C is able to perform a clear observation is an observation range by the high magnification image pickup unit 39 of the high magnification observation probe portion 42C.

According to this setting, if the area of interest 17a for which a high magnification observation is necessary is recognized in the observation area 17 during an observation by the normal observation image pickup unit 27, a magnification observation operation by the high magnification image pickup unit 39 can be performed by approaching the high magnification observation probe portion 42C toward the area of interest 17a.

The observation range by the high magnification image pickup unit 39 is, for example, 1 mm × 1 mm or less and a resolution thereof is 5 µm or less.

The marker supply pipeline 44 provided at the high magnification observation probe portion 42C is connected to the marker supply control portion 66. A marker is supplied to the marker supply pipeline 44 from the marker supply control portion 66 at the time of marking. The marker is discharged from the exhaust port 44a of the marker supply pipeline 44 with the distal end surface 35Ca of the high magnification observation probe portion 42C being in contact with the surface of the area of interest 17a in the observation area 17. As a result, marking can be performed onto the area of interest 17a.

As described above, the endoscope 2C has the forceps channel 23. The coloring matter spray instrument 74 is inserted into the channel 23 so that a coloring matter can be locally sprayed to an area to be magnified and observed by the high magnification image pickup unit 39.

The coloring matter spray instrument 74 has a syringe 75 containing a coloring matter solution 78 in which a coloring matter is dissolved, and a flexible tube 76 connected to the syringe 75 and capable of being inserted into the forceps channel 23. The coloring matter spray instrument 74 feeds the coloring matter solution 78 to a distal end side thereof through the flexible tube 76 by pushing a piston of the syringe 75. Then, the coloring matter solution 78 is jetted through a nozzle portion 77 attached to a distal end of the flexible tube 76 so that the coloring matter can be sprayed to a desired area 79 during the observation by the endoscope 2C.

After spraying the coloring matter solution 78, for example, the syringe 75 on a hand side is replaced with a syringe containing water or the like, water or the like is sprayed, and the coloring matter solution 78 that has spread the coloring matter is washed away.

An area stained by spraying the coloring matter is colored by the coloring matter to be displayed in an endoscope image displayed in the endoscope image displaying area 63 of the monitor 6. When the stained area is observed by the high magnification image pickup unit 39, the stained area is displayed as a magnification observation image in the magnification observation image displaying area 64 of the monitor 6.

As shown in Fig. 1, the monitor 6 has the endoscope image displaying area 63 and the magnification observation image displaying area 64 in which an endoscope image and a magnification observation image are respectively displayed adjacent to each other. In the present embodiment, the high magnification observation probe portion 42C is integrally attached to the distal end portion 18 of the endoscope 2C. Accordingly, a magnification observation location of the high magnification image pickup unit 39 in the endoscope image displaying area 63 is defined.

An endoscopy method according to the endoscope observation apparatus 1C of the present embodiment is described with reference to Fig. 10.

A body cavity is observed with a normal magnification by the endoscope 2C and the area 79 to be magnified and observed is identified as the area of interest 17a. Then, a coloring matter solution is sprayed to the area 79 by the coloring matter spray instrument 74 for performing a spraying of a coloring matter. After that, a process for washing away the sprayed coloring matter is performed.

Next, an optical window portion of the distal end surface of the high magnification observation probe portion 42C is pressed against (brought into contact with) the area 79 stained by spraying the coloring matter, or an area having the sprayed coloring matter washed away and removed, that is, the area of interest 17a.

In the present embodiment, the high magnification observation probe portion 42C is fixed to and supported by the distal end portion 18 of the endoscope 2C. An optical axis of the image pickup unit 39 is positioned so as to be, for example, on a horizontal line passing through the optical axis of the normal observation objective lens system 28 in the endoscope 2C. Because of this, an operation of pressing the distal end surface 35Ca of the high magnification observation probe portion 42C against the surface of the area of interest 17a can be easily performed.

The signal switching device 72 is switched, for example, by one frame period or the like of a video signal from the video processor 5 and an endoscope image and a magnification observation image can be displayed adjacent to each other on the monitor 6. In this state, the area of interest 17a to be magnified and observed is image-picked up with a high magnification by the high magnification objective lens system 40 and the CCD 41 of the high magnification image pickup unit 39, and displayed on the monitor 6 as a magnification observation image (a high magnification observation step).

Additionally, data of these images are recorded in the recording apparatus 7 shown in Fig. 9.

It is possible to mark the surface of the area of interest 17a in the observation area 17 through the marker supply pipeline 44 of the high magnification observation probe portion 42C before, after or during the high magnification observation. That is, a marker is supplied to the marker supply pipeline 44 from the marker supply control portion 66 to be discharged from the exhaust port with the distal end surface 35Ca of the high magnification observation probe portion 42C being in contact with the surface of the area of interest 17a. As a result, marking can be performed close to the area of interest 17a (a marking step).

The endoscope 2C of the present embodiment has an effect similar to the endoscope 2 of the first embodiment. In particular, the high magnification observation probe portion 42C with the image pickup unit 39 is fixed and located to the distal end portion 18 of the endoscope 2C projecting by the predetermined projection distance L1. Therefore, if a high magnification observation or marking to an area of interest is performed, the distal end surface 35Ca of the high magnification observation probe portion 42C may be simply brought into contact with the surface of the area of interest 17a. Thus, the observation or the marking operation described above can be performed more easily and reliably.

The present invention is not limited to the respective embodiments and various modifications can be made. Further, each of the embodiments includes an invention in various stages and various inventions can be obtained by properly combining a plurality of components to be disclosed.

This application is based upon and claims the benefit of priority from the prior Japanese Patent Application No. 2006-152597, filed on May 31, 2006 in Japan.

## Claims

1. An endoscope (2) comprising:
an insertion portion (10) capable of being inserted into a body cavity;
first observation means (27) having a normal magnification and provided at a distal end portion (35) of the insertion portion (10);
a contact portion (35a) projected from a distal end surface of the distal end portion (35) and capable of being in contact with a subject;
marking means (44) provided at the contact portion (35a) and configured to apply a marker to the subject with which the contact portion (35a) is in contact;
**characterised by** second observation means (42) provided at the contact portion (35a) and capable of an optical high magnification observation.

2. The endoscope (2) according to claim 1, wherein an objective lens (40) of the second observation means (42) is provided at the contact portion (35a) and the marking means (44) is provided adjacent to the objective lens (40).

3. The endoscope (2) according to claim 2, wherein the marking means (44) is provided around the objective lens (40) forming a ring shape.

4. The endoscope (2) according to claim 1, wherein the marking means (44) is configured to discharge or transfer a fluid, solid or gel marker including a coloring matter to apply the marker to the subject.

5. The endoscope (2) according to claim 1, wherein the marking means (44) is configured to perform marking by color-changing an observation area by heating.

6. The endoscope (2) according to claim 1, wherein the marking means (44) is configured to perform marking by applying a trace to the observation area by putting a needle in and out.

## Patentansprüche

1. Endoskop (2), das umfasst:
einen Einführabschnitt (10), der dazu eingerichtet ist, in einen Körperhohlraum eingeführt zu werden;
ein erstes Beobachtungsmittel (27), das eine normale Vergrößerung hat und an einem distalen Endabschnitt (35) des Einführabschnitts (10) angeordnet ist;
einen Kontaktabschnitt (35a), der von einer distalen Endoberfläche des distalen Endabschnitts (35) vorsteht und dazu in der Lage ist, in Kontakt mit einem Subjekt zu sein;
ein Markierungsmittel (44), das an dem Kontaktabschnitt (35a) vorgesehen ist und dazu eingerichtet ist, eine Markierung an dem Subjekt anzubringen, mit dem der Kontaktabschnitt (35a) in Kontakt ist;
**gekennzeichnet durch** ein zweites Beobachtungsmittel (42), das an dem Kontaktabschnitt (35a) vorgesehen ist und zu einer optischen Beobachtung mit einer hohen Vergrößerung in der Lage ist.

2. Endoskop (2) gemäß Anspruch 1, bei dem eine Objektivlinse (40) des zweiten Beobachtungsmittels (42) an dem Kontaktabschnitts (35a) vorgesehen ist und das Markierungsmittel (44) benachbart zu der Objektivlinse (40) angeordnet ist.

3. Endoskop (2) gemäß Anspruch 2, bei dem das Markierungsmittel (44) um die Objektivlinse (40) vorgesehen ist und eine Ringform bildet.

4. Endoskop (2) gemäß Anspruch 1, bei dem das Markierungsmittel (44) dazu eingerichtet ist, eine flüssige, eine feste oder eine gelförmige Markierung, die ein Färbemittel umfasst, abzugeben oder zu übertragen, um die Markierung an dem Subjekt anzubringen.

5. Endoskop (2) gemäß Anspruch 1, bei dem das Markierungsmittel (44) dazu eingerichtet ist, eine Markierung durch eine Änderung der Farbe eines Beobachtungsgebiets durch Erhitzen durchzuführen.

6. Endoskop (2) gemäß Anspruch 1, bei dem das Markierungsmittel (44) dazu eingerichtet ist, eine Markierung durch Aufbringen einer Spur auf das Beobachtungsgebiet durch Einführen und Herausziehen einer Nadel durchzuführen.

## Revendications

1. Endoscope (2) comprenant :
une partie d'insertion (10) capable d'être insérée dans une cavité de corps ;
un premier moyen d'observation (27) ayant un grossissement normal et prévu au niveau d'une partie d'extrémité distale (35) de la partie d'insertion (10) ;
une partie de contact (35a) faisant saillie depuis une surface d'extrémité distale de la partie d'extrémité distale (35) et capable d'être en contact avec un sujet ;
un moyen de marquage (44) prévu au niveau de la partie de contact (35a) et configuré pour appliquer un marqueur sur le sujet avec lequel la partie de contact (35 a) est en contact ;
**caractérisé par** un deuxième moyen d'observation (42) prévu au niveau de la partie de contact (35a) et capable d'une observation avec un grossissement optique élevé.

2. Endoscope (2) selon la revendication 1, dans lequel une lentille d'objectif (40) du deuxième moyen d'observation (42) est prévue au niveau de la partie de contact (35a) et le moyen de marquage (44) est prévu adjacent à la lentille d'objectif (40).

3. Endoscope (2) selon la revendication 2, dans lequel le moyen de marquage (44) est prévu autour de la lentille d'objectif (40) formant une forme annulaire.

4. Endoscope (2) selon la revendication 1, dans lequel le moyen de marquage (44) est configuré pour évacuer ou transférer un marqueur liquide, solide ou en gel incluant une matière colorante pour appliquer le marqueur sur le sujet.

5. Endoscope (2) selon la revendication 1, dans lequel le moyen de marquage (44) est configuré pour réaliser un marquage par changement de couleur d'une zone d'observation en chauffant.

6. Endoscope (2) selon la revendication 1, dans lequel le moyen de marquage (44) est configuré pour réaliser un marquage en appliquant une trace sur la zone d'observation en entrant et sortant une aiguille.
